# EUROPEAN PATENT SPECIFICATION

(11) **EP 3 244 821 B1**
(45) Date of publication and mention of the grant of the patent: **08.03.2023**
(21) Application number: 15878202.9
(22) Date of filing: 13.01.2015
(51) Int. Cl.: A61B 18/18, A61B 18/14

(54) **PRECISION BLADE ELECTROSURGICAL INSTRUMENT**
ELEKTROCHIRURGISCHES INSTRUMENT MIT PRÄZISIONSKLINGE
INSTRUMENT ÉLECTROCHIRURGICAL À LAME DE PRÉCISION

(43) Date of publication of application: 22.11.2017
(73) Proprietor: Megadyne Medical Products, Inc., Draper, UT 84020 (US)
(72) Inventor: BORGMEIER, Paul R., Salt Lake City, UT 84124 (US); WALTER, Brian J., Draper, UT 84020 (US); GREEP, Darcy W., Herriman, UT 84096 (US)
(74) Representative: Carpmaels & Ransford LLP
(86) International application number: PCT/US2015/011127
(87) International publication number: WO 2016/114760

(56) References cited:
- WO-A1-99/40858
- WO-A2-2005/046739
- US-A- 6 105 581
- US-A1- 2007 093 811
- US-A1- 2008 027 428
- US-A1- 2012 215 217
- US-B2- 7 736 361
- US-B2- 8 439 910
- US-B2- 8 500 727

## Description

### BACKGROUND

### 1. Technical Field

This disclosure relates to electrosurgical devices. More particularly, the disclosure relates to electrosurgical electrodes for use in performing electrosurgery.

### 2. The Relevant Technology

Modern surgical techniques frequently involve cutting tissue and/or and cauterizing cut tissue to coagulate or stop bleeding encountered during performance of a surgical procedure. In the area of electrosurgery, medical procedures of cutting tissue and/or cauterizing leaking blood vessels are performed by utilizing radio frequency (RF) electrical energy. The RF energy is produced by a wave generator and transmitted to a patient's tissue through a hand-held electrode that is operated by a surgeon. The hand-held electrode delivers an electrical discharge to cellular matter of the patient's body adjacent to the electrode. The discharge causes the cellular matter to heat up in order to cut tissue and/or cauterize blood vessels. For a historical perspective and details of such techniques, reference is made to United States Patent No. 4,936,842, issued to D'Amelio et al., and entitled "Electroprobe Apparatus," the entire disclosure of which is incorporated herein by this reference.

Electrosurgery is widely used and offers many advantages including the use of a single surgical instrument for both cutting and cauterizing/coagulating. Typical monopolar electrosurgical systems have an active electrode, such as in the form of an electrosurgical instrument having a hand piece and a conductive electrode (e.g., tip or blade), which is applied by the surgeon to the patient at the surgical site to perform surgery, and a return electrode to connect the patient back to the generator, thus completing the circuit. The electrode of the electrosurgical instrument produces a high density RF current at the point of contact with the patient in order to produce a surgical effect of cutting or coagulating the tissue. The return electrode carries the same RF current provided to the electrode or tip of the electrosurgical instrument, after the RF current passes through the patient, by completing the circuit, thus providing a path back to the electrosurgical generator.

A variety of proposals have heretofore been embodied in existing electrosurgical implements. Examples of such proposals include those set forth in: U.S. Pat. No. 4,534,347 to Leonard S. Taylor; U.S. Pat. No. 4,674,498 to Peter Stasz; and U.S. Pat. No. 4,785,807 to G. Marsden Blanch. The former two of the foregoing patents illustrate implements having sharpened exposed edges (e.g., knife-blade like geometries) which are employed to perform conventional mechanical cutting of tissue. The latter of the patents sets forth an unsharpened blade which has been entirely coated with an insulating layer so that cutting is performed by electrical energy capacitively transferred across the insulating layer rather than by conventional mechanical action.

It is widely accepted that in electrosurgery, "cutting" is accomplished when energy transfer is sufficient to cause water in tissue cells to boil, thus rupturing the cell membranes by internal rather than external forces. A high level of energy is required to effectuate such electrosurgical cutting, leading to a corresponding high temperature of the electrode. The high temperatures involved in electrosurgery can cause thermal necrosis of the tissue adjacent the electrode. The longer tissue is exposed to the high temperatures involved with electrosurgery, the more likely it is that the tissue will suffer thermal necrosis. Thermal necrosis of the tissue can decrease the speed of cutting the tissue and increase post-operative complications, eschar production, and healing time, as well as increasing incidences of heat damage to tissue away from the cutting site.

While the Blanch proposals have constituted an important advance in the art and have found wide-spread acceptance in the field of electrosurgery, there has been a continuing need for further improvement in electrosurgery to increase the precision of cutting and reduce thermal necrosis, thereby decreasing healing time and post-operative complication, reducing eschar production, reducing incidence of heat damage to tissue away from the cutting site, and increasing the speed of cutting. In particular, traditional electrosurgical electrodes are not very precise in their application of energy and as a result, the thermal spread and tissue damage they create can be problematic. Likewise, traditional electrodes are not effectively maneuverable in small, compact, or sensitive tissue locations. For this reason, traditional monopolar electrosurgical electrodes have not been highly effective in certain procedures or under certain conditions (e.g., neurological/spinal/cranial surgeries and pediatric procedures).

To overcome these and other disadvantages in the use of traditional electrosurgical electrodes for electrosurgery, electrosurgical needle electrodes have been employed with some degree of success. However, electrosurgical needles have certain limitations, especially in their dissection or cutting capabilities (e.g., long incisions or dissections along a plane) and maneuverability.

Accordingly, there are a number of disadvantages in conventional electrosurgical devices that can be addressed. Specifically, it would be advantageous to have an electrode that is highly maneuverable and adapted for dissecting or cutting along a tissue plane, and that limits unwanted tissue damage, reduces post-operative complications, increases the speed and precision of cutting, and facilitates quicker healing.

An electrosurgical instrument is known from WO2005/046739A2. Therein disclosed is an electrosurgical instrument including a main body having an outer, insulating layer. Insulating layer comprises a porous, insulating material, or coating, that has been sealed. The main body includes a flattened, distally-extending blade portion and a proximally extending cylindrical shaft portion. The blade portion has a reduced cross-section laterally outward, such reduction being made either continuously, for example, by tapering, or using one or more steps, to produce a relatively thin outer peripheral edge about at least a distal tip to define peripheral edge portion. In one arrangement, electrosurgical instrument comprises a blade portion having a thickness of about 5.08 mm ("0.020 inches"), a width of about 3.556 mm ("0.140 inches") and length of about 25.4 mm ("1 inch"). Peripheral edge portion has outer extreme edge thickness of about 0.0254 mm ("0.001 inches") or less.

Methods, systems, and devices for performing electrosurgical procedures are known from US8500727B2. Therein disclosed is an electrosurgical system comprising an electrosurgical electrode adapted to transmit electrical energy to patient tissue during an electrosurgical procedure, the electrode having a configuration that enhances rapid feedback used to automatically correct and regulate power levels applied to the electrode, the electrode comprising a plurality of working surfaces with differing lengths that are angled relative to one another, but wherein each working surface has a thickness in the range of about 0.254 mm to about 0.1270 mm that is sharpened to concentrate the electrical energy transmitted from the electrode to the patient tissue during an electrosurgical procedure so that concentration of the electrical energy to the patient tissue improves both speed with which the electrode is able to cut or cauterize the tissue while also reducing likelihood of tissue damage in tissue surrounding the cut or cauterized tissue, the improved speed with which the electrode is able to cut or cauterize tissue providing more rapid feedback that can be used to regulate power levels applied to the electrode, and the electrode having an overall thickness of about 0.4318 mm so that the electrode has a limited mass which limits the amount of latent heat the electrode is able to retain; and an electrosurgical wave generator electrically coupled to the electrode, the wave generator comprising; a power source for providing electrical energy to said electrode working surface at a specified frequency; a voltage sensor and a current sensor coupled between the power source and the electrode for sensing both voltage and current at the electrode at a sampling rate of about every 20 milliseconds in order to provide voltage and current feedback parameters from which the tissue impedance can be essentially continuously re-computed about every 20 milliseconds and then used to rapidly adjust power levels applied to the electrode during a cut or cauterize procedure to avoid application of excess power and tissue damage; and a processor programmed with executable instructions for processing the sensed voltage and current to recompute tissue impedance for each sample taken while cutting or cauterizing the tissue so that (i) as cutting or cauterizing the tissue is initiated, power to the electrode is initially rapidly increased up to a pre-determined tissue impedance level, and then as tissue impedance increases above the pre-determined tissue impedance level, the power to the electrode is rapidly reduced in inverse proportion to for the increases in tissue impedance.

US2008/027428A1 describes an electrosurgical cutting system that comprises an electrically conductive blade having an uninsulated cutting edge that is surrounded by an insulator and US6105581A describes an electrosurgical probe that includes one or more electrode terminals and a return electrode coupled to a high frequency voltage supply.

### BRIEF SUMMARY

In accordance with the present invention, there is provided an electrode as defined by claim 1. Dependent claims 2-6 disclose exemplary embodiments.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope, the scope of the invention being defined by the appended claims. The invention will be described and explained with additional specificity and detail through the use of the accompanying drawings in which:
Figure 1 illustrates a schematic view of an exemplary electrosurgical system according to an embodiment of the present disclosure;
Figure 2 illustrates a top plan view of an exemplary electrosurgical electrode, not according to the invention, for use with the electrosurgical system of Figure 1;
Figure 2A illustrates a cross-sectional view of the electrosurgical electrode of Figure 2;
Figure 3 illustrates a top plan view of another exemplary electrosurgical electrode not according to the invention;
Figure 3A illustrates a cross-sectional view of the electrosurgical electrode of Figure 3;
Figure 4 illustrates a perspective view of an exemplary electrosurgical electrode according to the present invention;
Figure 4A illustrates a cross-sectional view of the electrosurgical electrode of Figure 4;
Figures 5A-5H each illustrate a cross-sectional view of an exemplary electrosurgical electrode not according to the invention;
Figures 6A-6E each illustrate a cross-sectional view of an exemplary electrosurgical electrode not according to the invention;
Figures 7B-7C and 7E-7O each illustrate a cross-sectional view of an exemplary electrosurgical electrode not according to the invention; Figures 7A and 7D each illustrate a cross-sectional view of an exemplary electrosurgical electrode according to the invention; and
Figures 8A-8G each illustrate a cross-sectional view of an exemplary electrosurgical electrode not according to the invention.

### DETAILED DESCRIPTION

In accordance with the present invention, there is provided an electrode as defined by claim 1. Dependent claims 2-6 disclose exemplary embodiments..

In one or more other embodiments, an electrode can include one or more longitudinal side edges that have a thickness that is less than or equal to about 0.254mm (0.01 inches) (each comprising one longitudinal cutting edge) and one or more longitudinal side edges that have a thickness that is greater than about 0.254mm (0.01 inches) (each comprising two or more longitudinal cutting edges).

As used herein, "side edge," "longitudinal side edge," and similar terms refer to a transition area and/or structural feature between two major surfaces. Those skilled in the art will appreciate that even a continuous surface that wraps around from an arbitrary starting point back to said starting point can form two major surfaces (e.g., one on either side of the starting point). Similarly, a continuous surface that wraps around to bring one end thereof into proximity with another (opposing) end can form two major surfaces

As used herein, "cutting edge," "longitudinal cutting edge," and similar terms refer to a portion of an electrosurgical electrode that is structurally operable, adapted, and/or configured to, and/or capable of, dissecting a patient's tissue in, along, and/or through an anatomical or naturally occurring tissue plane or line of separation between tissue types, tissue borders, or structures. Those skilled in the art will appreciate that such dissection occurs along and/or over a distance (e.g., from a first position toward a second position). Furthermore, the electrosurgical dissection plane can comprise a straight line, curved line, angled line, circular line (where the second position corresponds to the first position after dissection along the distance), and/or combinations thereof.

One or more embodiments implement so called "precision technology," which limits the cross-sectional area of the electrode in combination with at least one or more edges arranged parallel to the longitudinal length of the electrode. In some embodiments, precision technology design principle(s) allows for the creation of electrodes capable of, adapted for, and/or configured for precise application of electrosurgical energy to tissue with enhanced maneuverability, reduced power requirements compared to some existing technologies, low thermal spread that reduces the amount of necrotic damage to the tissue surrounding the incision site compared to some existing technologies, and/or the ability to dissect readily along a plane and/or operate safely within a smaller, narrowed, restricted, constricted or localized area.

The ratio of cross-sectional area (A) to number of longitudinal cutting edges (E), in particular, is unexpectedly important to (or determinant of) one or more benefits of certain embodiments of the present disclosure. In one embodiment, the body can comprise (only) longitudinal side edge(s) having a thickness less than or equal to about 0.254mm (0.01 inches), one longitudinal cutting edge associated with each longitudinal side edge, and a cross-sectional area-to-number of longitudinal cutting edges ratio that is less than or equal to about 0.096774 mm²/E (0.000150 in²/E). In one or more other embodiments, the body can comprise a hybrid, combination, or mixture of (a) longitudinal side edge(s) having a thickness less than or equal to about 0.254mm (0.01 inches) (each having (only) one longitudinal cutting edge associated therewith), and (b) longitudinal side edge(s) having a thickness greater than about 0.254mm (0.01 inches) (each having at least two longitudinal cutting edges associated therewith).

Those skilled in the art will appreciate that the cross-sectional area of embodiments described herein includes the cross-sectional area of the body of the electrosurgical electrode. A variety of cross-sectional area measuring techniques are known in the art and contemplated herein. For instance, the cross-sectional area of a rectangular-shaped body can be determined by measuring and multiplying the (vertical and horizontal) dimensions thereof (e.g., base (or width) times height (or thickness)). Similarly, the cross-sectional area of a triangular-, trapezoidal-, rhomboidal-, and/or diamond-shaped body can be determined by measuring and multiplying the (vertical and horizontal) dimensions thereof and dividing by two (e.g., one-half base (or width) times height (or thickness)).

By way of illustration, if an electrode is constructed with a body having a first side edge thickness less than or equal to 0.254mm (0.01 inches), and a second side edge thickness greater than 0.254mm (0.01 inches), and a uniform, linear tapering between the two edges, the cross-sectional area of the body can be calculated based on the average of these two edge thicknesses (i.e., (first thickness + second thickness)/2). As indicated above, the cross-sectional area to cutting edge ratio for this configuration can be less than or equal to about 0.001 in²/E (i.e., (W x Tₐᵥₑ)/E ≤ 0.001 in²/E), 1 inch = 2.54 cm.

Without being bound to any theory or restricted to limitations as of the filing of this disclosure, those skilled in the art will also appreciate that manufacturing of electrosurgical electrodes may not allow for the formation of one or more true geometric shapes. In particular, a geometric corner, point, tangent, or intersection of sides, for example, while ideal, may not be attainable by available manufacturing processes or methods.

In at least one embodiment, the cross-sectional area can be determined by determining the cross-sectional area of one or more geometric or non-geometric shapes to which the cross-section of the body is closely related (or by which it is most closely approximated). Thus, the cross-sectional area of a substantially rectangular body can be estimated (or determined) by measuring and multiplying the (vertical and horizontal) dimensions thereof (e.g., base (or width) times height). In an alternative embodiment, a true cross-sectional area (e.g., accounting for the absence of small or even minute edge and/or corner roundings) can be determined and/or measured. For instance, modeling software or other measuring mechanisms can be utilized to determine the actual cross-sectional area occupied by the material(s) forming the body of the electrosurgical electrode. Thus, certain embodiments having one or more protrusions or other shaped features can have a true cross-sectional area determined accurately and/or precisely.

In other embodiments, the cross-sectional area can comprise the cross-sectional area circumscribed, encompassed, covered, and/or occupied by the (outer-most) dimensions, edges, protrusions, and/or surfaces of the body (e.g., the least, common dimension(s) and/or cross-sectional area of the body). For instance, the cross-sectional area can comprise the longest or outer-most dimension of the body in the x-plane times the longest or outer-most dimension of the body in the y-plane or other similar calculus.

The cross-sectional area of a body having one or more (convex) protrusions can include the cross-sectional area defined and/or circumscribed by the (combined) outer dimensions of the protrusion(s) and the body portion. Thus, any apparently recessed portions between the protrusions can contribute to the cross-sectional area of the body in some embodiments. The cross-sectional area of a body having one or more protrusions can also (or alternatively) comprise the cross-sectional area of the protrusion(s) plus the cross-sectional area of the body portion (without the protrusions). Thus, any apparently recessed portions between the protrusions may not contribute to the cross-sectional area of the body in some embodiments.

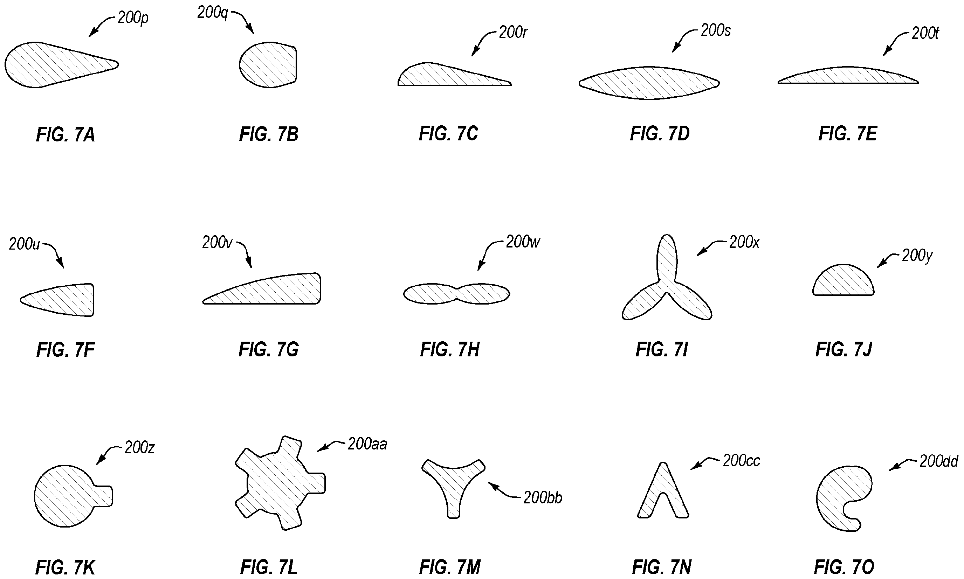

In at least one embodiment, a body having one or more concave portions can have a cross-sectional area determined by inclusion of the concave portion in the calculus. For instance, a substantially triangular body having a substantially triangular recessed portion therein (see e.g., Figure 7N) can have a cross-sectional area that includes the recessed portion as part of the body (i.e., as if there were no concave, recessed portion in the triangular-shaped body). Thus, a body having one or more concave portions can have a cross-sectional area comprising the area circumscribed by the body and one or more concave portions. In other embodiments, a body having no concave portions can have a cross-sectional area equal to the actual cross-sectional area of the material forming the body. Thus, a body having a convex (or entirely convex) body can have a cross-sectional area comprising the area occupied by the body (or suitable approximation thereof, as described above).

In at least one embodiment, the body can comprise an elongated body extending longitudinally between a first end and a second end. The first and second ends can be separated by a first length. Similarly, the one or more longitudinal side edges can extend a second length between the first end and the second end. In at least one embodiment, the first length and second length can be substantially equal such that one or more longitudinal side edges extend between the first end and the second end. In other embodiments, the second length can be shorter that the first length such that the one or more longitudinal side edges do not extend all the way between the first end and the second end.

In some embodiments, the thickness of the one or more longitudinal side edges and/or the at least one of the one or more longitudinal side edges can be measured between a first major surface and a second major surface (e.g., opposite the first major surface). In some embodiments, the at least one of the one or more longitudinal side edges is continuous with the first major surface and the second major surface such that (i) at least one of the two or more longitudinal cutting edges comprises a junction between the first major surface and the at least one of the one or more longitudinal side edges, and (ii) at least one of the two or more longitudinal cutting edges comprises a junction between the second major surface and the at least one of the one or more longitudinal side edges.

In an embodiment according to the invention, the body comprises a first major surface and a second major surface opposite the first major surface, (ii) first and second longitudinal side edges (disposed between the first major surface and the second major surface), at least one of the one or more longitudinal side edges having a thickness less than or equal to about 0.254 mm (0.01 inches), the at least one of the one or more longitudinal side edges having one longitudinal cutting edge, (iii) a cross-sectional area (e.g., disposed at least partially between the first major surface and/or the second major surface and/or the one or more longitudinal side edges), and (iv) a cross-sectional area-to-number of (effective) longitudinal cutting edges ratio less than or equal to about 0.096774 mm²/E (0.000150 in²/E).

In one or more embodiments, the at least one of the one or more longitudinal side edges is continuous with the first major surface and the second major surface such that the longitudinal cutting edge thereof comprises the junctions between the at least one of the one or more longitudinal side edges and the first and second major surfaces. Specifically, with a longitudinal side edge thickness less than or equal to about 0.254mm (0.01 inches), the at least one of the one or more longitudinal side edges does not produce and/or present more than one effective longitudinal cutting edge in certain embodiments. Hence, the at least one of the one or more longitudinal side edges has one longitudinal cutting edge in some embodiments.

In some embodiments, the cross-sectional area of the body can have a cross-sectional height between the first major surface and the second major surface. The cross-sectional height can be greater than about 0.254mm (0.01 inches). Furthermore, at least one of the one or more major surfaces (e.g., the first major surface and/or the second major surface) can include one or more tapered portions (e.g., extending from a portion of the at least one of the one or more major surfaces to the one or more longitudinal side edges (e.g., the at least one of the one or more longitudinal side edges (having one longitudinal cutting edge)) such that the thickness of the one or more longitudinal side edges is less than or equal to about 0.254mm (0.01 inches)). In certain embodiments, the one or more tapered portions can allow the cross-sectional height of the body to be greater than about 0.254mm (0.01 inches) and the thickness of the one or more longitudinal side edges to be less than or equal to about 0.254mm (0.01 inches). Thus, a hybrid configuration can be formed by one or more tapered portions.

As indicated above, in some embodiments, the one or more longitudinal side edges can include a plurality of longitudinal side edges (e.g., disposed between the first major surface and the second major surface and extending at least one length between the first end of the body and the second end of the body). At least one of the plurality of longitudinal side edges can have a thickness less than or equal to about 0.254mm (0.01 inches) (e.g., between the first major surface and the second major surface).

In some embodiments, the one or more tapered portions can include a plurality of tapered portions (e.g., extending from a portion of at least one of the first major surface and the second major surface to one or more longitudinal cutting edges (e.g., of the plurality of longitudinal cutting edges)). For instance, an embodiment can include a plurality of tapered portions extending from a portion of the first major surface to respective longitudinal side edges of the plurality of longitudinal side edges.

Another embodiment can include one or more tapered portions extending from a portion of the first major surface to one or more of the plurality of longitudinal side edges and one or more tapered portions extending from a portion of the second major surface to one or more of the plurality of longitudinal side edges. Another embodiment can include a plurality of tapered portions extending from a portion of the first major surface to respective longitudinal side edges of the plurality of longitudinal side edges and one or more tapered portions extending from a portion of the second major surface to one or more of the plurality of longitudinal side edges. Another embodiment can include a plurality of tapered portions extending from a portion of the first major surface to respective longitudinal side edges of the plurality of longitudinal side edges and a plurality of tapered portions extending from a portion of the first major surface to respective longitudinal side edges of the plurality of longitudinal side edges.

In certain embodiments, the one or more longitudinal side edges can be continuous with the first major surface and/or the second major surface. Furthermore, the electrode can comprise a tip (e.g., disposed at the first end of the body). The tip can also be disposed at least partially between the first major surface and the second major surface and/or continuous with one or more of the first major surface, the second major surface, and the one or more longitudinal side edges. The tip can also have a blunt configuration, a pointed configuration, a rounded configuration, an angled configuration, or any other suitable configuration.

One or more embodiments can include an insulating coating (e.g., covering at least a portion of the body). The insulating coating can be provided with or at a thickness sufficient to ensure transmission of the radio frequency electrical energy from the body to the tissue. The insulating coating can comprise or be a non-stick coating, as known in the art. The insulating coating can be formed of or comprise a material such as Polytetrafluoroethylene (PTFE), silicone, ceramic, glass, fluorinated hydrocarbon, diamond, a high temperature polymer, a hydrophilic polymer, a capacitor dielectric, and/or combinations thereof.

In at least one embodiment, the cross-sectional area of the body can have a cross-sectional width (e.g., between opposing side edges). In one embodiment according to the invention, the cross-sectional width is less than or equal to about 1.397 mm (0.055 inches). The cross-sectional area can also have a cross-sectional height (e.g., between the first major surface and the second major surface). The cross-sectional height can be less than or equal to 0.254mm (0.01 inches) in some embodiments. Alternatively, the cross-sectional height can be greater than about 0.254mm (0.01 inches) in some embodiments.

Furthermore, the cross-sectional area can have a cross-sectional shape or configuration. According to the invention, the cross-sectional shape is lenticular.

One will appreciate that manufacturing restraints may not permit a formation of the perfectly and/or geometrically "pointed" corners required to form one or more of the aforementioned configurations. Regardless, those skilled in the art will appreciate that cross-sectional configurations can comprise at least partially rounded corners and still comprise the cross-sectional shape or configuration recited.

In at least one embodiment, the electrode can be adapted for use in performing electrosurgical operative procedures at a power level that is reduced by at least, about, or greater than 5%, 10%, 15%, 20%, 25%, 30%, 35%, 40%, 45%, 50%, 55%, 60%, 65%, 70%, 75%, 80%, 85%, 90%, 95%, 1/4, 1/3, 1/2, 2/3, 3/4 or more than the typical power level used for a standard electrosurgical blade electrode in the same or similar tissue type or location. For example, a typical power level of 30 watts (or J/s) used for general surgical procedures may be reduced to 10 watts when using a precision electrode to accomplish the desired result, with improved thermal outcome and improved user maneuverability. In addition, the electrode can be adapted for a specific use in performing electrosurgical operative procedures at a power level that is reduced by at least, about, or greater than 5%, 10%, 15%, 20%, 25%, 30%, 35%, 40%, 45%, 50%, 55%, 60%, 65%, 70%, 75%, 80%, 85%, 90%, 95%, 1/4, 1/3, 1/2, 2/3, 3/4 or more compared to an electrosurgical blade electrode having a cross-section area-to-cutting edge ratio (a) greater about 0.0004 in²/E, (b) greater than about 0.000150 in²/E, and/or (c) greater than about 0.001 in²/E (e.g., compared to the power level typically used in the same or similar tissue type or location for an electrode with a ratio greater than the above) (1 inch = 2.54 cm). Thus, embodiments of the present disclosure allow significantly lower power transmission through tissues, thereby making such electrode embodiments readily useful and applicable, especially in sensitive areas or tissues prone to thermal injury, that do not typically benefit from the use of electrosurgery.

Described herein is a method, not according to the invention, of using an electrosurgical electrode (e.g., as described above) for performing an electrosurgical operative procedure. The electrode can be configured according to one or more of the embodiments described herein. In certain embodiments, the method can comprise (i) contacting the tissue with at least one of the one or more longitudinal cutting edges while energizing the electrode with radio-frequency electrical energy communicated from the generator (e.g., at an electrical power level at least 2/3 less than typical electrosurgical settings), thereby severing the tissue, (ii) advancing the energized electrode a certain distance, thereby electrosurgically dissecting the tissue along the plane extending from a first location of the tissue to a second location of the tissue, (iii) removing the at least one of one or more longitudinal cutting edges from contact with the patient tissue, (iv) identifying one or more patient sites needing coagulation, and/or (iv) cauterizing one or more patient sites needing coagulation by contacting the patient site needing coagulation with at least a part of the first major surface or the second major surface and energizing the electrode with radio-frequency electrical energy (e.g., at an electrical power level at least 2/3 less than typical electrosurgical settings) while contacting the one or more patient sites needing coagulation with at least a part of the first major surface or the second major surface, or spray coagulating the tissue from the surfaces and/or edges of the electrode.

In some embodiments, the method (which is not according to the invention) can include contacting the tissue with a tip disposed at the first end of the body (e.g., between the first major surface and the second major surface) while energizing the electrode with radio-frequency electrical energy communicated from the generator (e.g., at an electrical power level at least 2/3 less than typical electrosurgical settings), thereby severing the tissue. Furthermore, the method can include advancing the electrode while energizing the electrode with radio-frequency electrical energy communicated from the generator at an electrical power level at least 2/3 less than typical electrosurgical settings, thereby electrosurgically dissecting at least a portion of the tissue along the plane extending from the first location of the tissue to the second location of the tissue.

In at least one embodiment, the electrosurgical operative procedure (which is not according to the invention) can include a neurological surgery or procedure, a spinal surgery or procedure, a cranial surgery or procedure, and/or a pediatric surgery or procedure. In certain embodiments, advancing the energized electrode can comprise one or more changes in direction during dissection or while creating an incision. For instance, advancing the energized electrode can comprise a smooth, rounded or sharp, angled change in direction to the right, to the left, upward, or downward (e.g., while advancing the energized electrode along the dissection/incision path or plane).

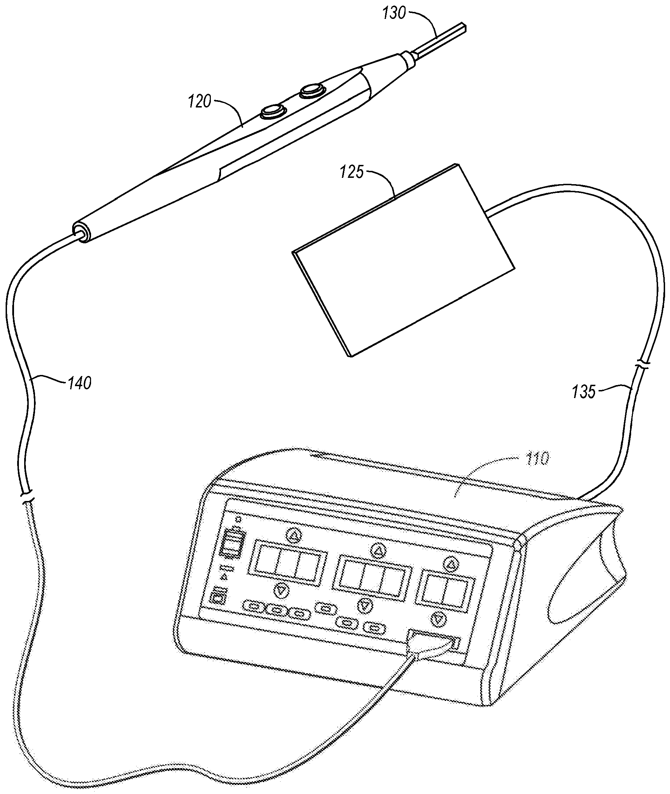

Turning now to the figures, Figure 1 and the corresponding discussion are intended to provide a brief, general description of an exemplary electrosurgical system in which one or more embodiments of the present disclosure can be implemented. Specifically, electrosurgical system 100 is illustrated, which includes a wave generator 110, a cable or cord 140, a hand-held instrument or hand piece 120, an electrosurgical electrode 130, a return electrode 125, and a cable or cord 135. Generator 110, in a preferred embodiment, is an RF wave generator. Accordingly, a surgeon or other user can use electrosurgical system 100 during surgical or other procedures to cut patient tissue and/or cauterize blood vessels of the patient's tissue.

In an illustrative electrosurgical procedure, RF electrical energy is produced by a wave generator, such as wave generator 110. The RF electrical energy is transferred to electrode 130 via hand piece 120, which is electrically coupled to wave generator 110 via cord 140. Those skilled in the art will appreciate that wave generator 110 can include a high-frequency oscillator and amplifier(s) to generate an RF electrical energy wave that can be used to cut tissue and/or cauterize blood vessels during electrosurgery.

The RF electrical energy wave powers hand piece 120 in order to produce an electrical discharge from electrode 130 to the patient tissue. The electrical discharge can cause heating of cellular matter of patient tissue that is in direct (or indirect, e.g., close or extremely close) contact with electrode 130. In particular, the energy transfer can be sufficient to cause water in tissue cells to boil, thus rupturing the cell membranes, thereby electrosurgically cutting, severing, and/or dissecting the patient tissue. In at least one embodiment, electrosurgically cutting, severing, and/or dissecting does not involve a significant amount or degree of mechanical cutting, severing, and/or dissecting. In some embodiments, electrode 125 provides a return electrical path via cord 135 to wave generator 110 for charge that dissipates into surrounding tissue of the patient's body. One will appreciate that terms such as cutting, severing, and/or dissecting can be used interchangeably herein to reflect various types of surgical tissue separation, etc.

In some embodiments, during electrosurgery, electrical discharge from electrode 130 can be used to independently or concurrently cut and cauterize. For instance, a constant sinusoidal wave supplied by wave generator 110 and transmitted to hand piece 120 can allow electrode 130 to cut through tissue of the patient's body. Alternatively, a dampened wave supplied by wave generator 110 and transmitted to hand piece 120 can allow electrode 130 to cauterize leaking blood vessels. In at least one embodiment, a combination of the constant sinusoidal wave and the damped wave can be supplied by wave generator 110 to hand piece 120 for allowing electrode 130 to concurrently cut and cauterize, thereby minimizing tissue trauma and blood loss during the surgical procedure. For instance, in one or more embodiments, an alternating transmission of constant sinusoidal wave and/or damped wave can be supplied by wave generator 110 to hand piece 120 for allowing electrode 130 to concurrently cut and cauterize, thereby minimizing tissue trauma and blood loss during the surgical procedure.

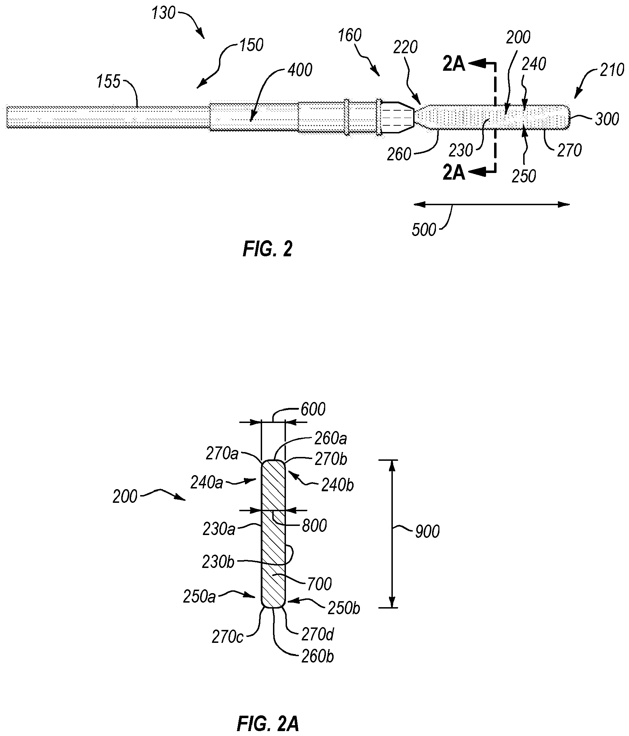

By way of introduction, Figures 2, 3, and 4 illustrate an exemplary assortment of (interchangeable) electrodes 130, or various features, designs, and/or configurations thereof. Figures 2A, 3A, 4A, and 5A-8G illustrate an exemplary assortment of cross-sectional configurations for (a body of) an electrosurgical electrode 130.

Figure 2, for example, illustrates a generic electrode, not according to the invention, for illustrative purposes. As illustrated in Figure 2, the electrode 130 has (i) a connection end 150 configured to be coupled to a hand piece 120 to allow RF electrical energy generated by wave generator 110 to be transmitted through hand piece 120 to electrode 130, and (ii) a working end 160 configured to contact patient tissue and apply the electrical discharge to the patient tissue. In at least one embodiment, working end 160 and connection end 150 can comprise opposing ends of electrode 130.

One will appreciate, however, that electrode 130 need not comprise an elongated configuration as illustrated in Figure 2. For instance, electrode 130 can have a curved, angled, circular, flat, broad, or other configuration without departing from the scope of this disclosure. Indeed, in some embodiments, electrode 130 can have any configuration suitable for use in an electrosurgical procedure.

In some embodiments, connection end 150 can include a connection member 155 configured to be coupled to a hand piece 120. The lengths of connection end 150 and/or connection member 155 thereof can vary depending on the specific type of electrode and/or the type of procedure for which the electrode is used. For instance, the lengths of the connection ends may range from about 6.35 cm to about 48 cm in certain embodiments. In various embodiments, the lengths of the connection ends can be about 6.35 cm, 6.9 cm, 10.16 cm, 15.24 cm, 33 cm, 45 cm, and 48 cm, respectively. It will be appreciated that the lengths of the connection ends can be any suitable lengths and are not intended to limit the scope of the present disclosure.

Electrode 130 can also include a sleeve or coating 400 (e.g., that surrounds at least a portion of the electrode 130) to act as an insulator, provide protection, and/or facilitate holding of the electrode 130 by hand piece 120. For example, an insulative material can be applied to a portion of the working end 160 of the electrode 130 in order to provide an insulative barrier between a portion of the working end 160 and a patient tissue.

In one embodiment, the insulative material can be applied around at least a portion of the working end 160 of the electrode 130 (e.g., leaving part (e.g., only a small part) of the electrode tip exposed for use during electrosurgery). For example, the insulative material can cover the entire working end except for about 0.3 cm at an end of the electrode 130 or working end 160 thereof. The exposed portion can then be used to perform electrosurgery without electrical discharge between the rest of the electrode 130 (or working end 160 thereof) and the patient's tissue. In an alternative embodiment, the insulative material can leave a larger portion of the working end 160 of the electrode 130 exposed.

In some embodiments, the coating can comprise PTFE, fluoropolymer, Polyolefin, ceramic, PARYLENE, or combinations thereof. Such coatings can be applied, for example, to a portion of the working end 160 of the electrode 130 in order to provide an insulative barrier between a portion of the working end 160 and a patient's tissue.

The working end 160 of electrode 130 can also include a body 200 configured to contact patient tissue and apply the electrical discharge to the patient's body. Body 200 (and/or other portions of electrode 130) can comprise, include, and/or be formed of a conductive material. For instance, the conductive material can include stainless steel or other non-corrosive materials. Certain embodiments can also or alternatively include, for example, brass, nickel, aluminum, titanium, copper, silver, gold, other types of steel, or alloys (thereof). Some embodiments can also include non-metallic conductive substances (e.g., provided that they possess the inherent qualities of stability and integrity sufficient to meet the desired requisites), such as certain conductive plastics.

As will be discussed in further detail below, the body 200 of electrode 130 can have an elongated design or configuration. For instance, body 200 can include a first end 210 and a second end 220 (e.g., separated by a length identical or similar to length 500). One will appreciate, however, that body 200 need not comprise an elongated configuration as illustrated in Figure 2. For instance, body 200 can have a curved, angled, circular, flat, broad, or other configuration without departing from the scope of this disclosure. Indeed, in some embodiments, body 200 can have any configuration suitable for use in an electrosurgical procedure.

In at least one embodiment, electrode 130 can optionally include at least one tip 300 (e.g., disposed on, about, and/or adjacent to first end 210 of body 200). For instance, in some embodiments, body 200 can include or comprise tip 300. In certain embodiments, first end 210 can include tip 300 (e.g., tip 300 can extend from first end 210). Similarly, second end 220 can be connected, attached, and/or adjacent to (and/or extend from) connection end 150 or connection member 155 thereof. One will appreciate, however, that designations corresponding to first and/or second are illustrative only and not intended to limit the scope of this disclosure. Thus, in some embodiments, first end 210 can be connected, attached, and/or adjacent to (and/or extend from) connection end 150 or connection member 155 thereof.

Similarly, one or more tips 300 can be disposed at other locations and/or positions about electrode 130, working end 160, and/or body 200. For instance, body 200 can include one or more tips 300 disposed (longitudinally) thereon and/or forming one or more peaks along body 200. As will be discussed in further detail below, tip 300 can have or comprise any configuration, including one or more various design configurations, suitable for one or more particular electrosurgical procedures.

Body 200 can further include at least one major surface 230 and at least one side edge 260. In some embodiments, major surface 230 can include a first side 240 and/or (an opposing) second side 250. In at least one embodiment, side edge(s) 260 can be positioned at or adjacent to first side 240 and/or second side 250 of major surface 230. Furthermore, a longitudinal side edge 260 can extend a length 500 (e.g., between the first end 210 and the second end 220 of an elongated body 200).

In at least one embodiment, side edge 260 can comprise one or more cutting edges 270. For instance, side edge 260 can be continuous with major surface 230 such that cutting edge 270 comprises (or is formed at) the junction between major surface 230 and side edge 260. Similarly, the junction between major surface 230 and side edge 260 can form cutting edge 270.

As illustrated in Figure 2A, body 200 can include a first major surface 230a (having a first side 240a and second side 250a) and a second major surface 230b (having a first side 240b and second side 250b). First (longitudinal) side edge 260a can be positioned at or adjacent to (or between) first side 240a of first major surface 230a and first side 240b of second major surface 230b. Similarly, second (longitudinal) side edge 260b can be positioned at or adjacent to (or between) second side 250a of first major surface 230a and second side 250b of second major surface 230b.

Furthermore, one or more longitudinal side edge(s) (e.g., longitudinal side edge 260a and/or 260b) can comprise a thickness 600 (e.g., between first major surface 230a and second major surface 230b). For instance, longitudinal side edge 260a can have a thickness 600 between first major surface 230a at or adjacent to first side 240a thereof and second major surface 230b at or adjacent to first side 240b thereof. One will appreciate that longitudinal side edge 260b can be identically, similarly, or differently configured. For instance, longitudinal side edge 260a can have a thickness greater than the thickness of longitudinal side edge 260b, or vice versa.

In at least one embodiment, (the magnitude of) thickness 600 can contribute to and/or determine the number of cutting edges (E) 270 included in body 200. For instance, those skilled in the art will appreciate that in some embodiments once thickness 600 becomes sufficiently large, the transition between first major surface 230a and second major surface 230b (or between a major surface 230 and a side edge 260), comprises, accommodates, forms, and/or permits the formation of two or more cutting edges 270. One will appreciate, however, that side edge 260 can comprise a single cutting edge 270 (e.g., even where thickness 600 is greater than about 0.254mm (0.01 inches)) in some embodiments.

In at least one embodiment, a thickness 600 (of side edge 260 and/or between major surfaces 230) greater than about 0.254mm (0.01 inches) can comprise, accommodate, form, and/or permit the formation of two or more cutting edges 270. For instance, a thickness 600 of (greater than or equal to) about 0.011, 0.012, 0.0125, 0.015, 0.0175, 0.0195, 0.0195, 0.02, and/or 0.05 inches (or any value or range of values therebetween) can comprise, accommodate, form, and/or permit the formation of two or more (effective) cutting edges 270. Thus, embodiments of the present disclosure can include a thickness of (i) about, (ii) greater than or equal to about, (iii) at least about, and/or (iv) between about 0.011, 0.012, 0.0125, 0.015, 0.0175, 0.0185, 0.0195, 0.02, 0.05, and 0.075 inches (or any value or range of values therebetween, 1 inch = 2.54 cm.

As illustrated in Figure 2A, for example, body 200 can comprise four cutting edges 270a, 270b, 270c, and 270d. Specifically, the junction or transition between side edge 260a and first major surface 230a (at first side 240a thereof) can comprise, accommodate, form, and/or permit the formation of a first (distinct and/or effective) cutting edge 270a. Similarly, the junction or transition between side edge 260a and second major surface 230b (at first side 240b thereof) can comprise, accommodate, form, and/or permit the formation of a second (distinct and/or effective) cutting edge 270b. Likewise, the junction or transition between side edge 260b and first major surface 230a (at second side 250a thereof) can comprise, accommodate, form, and/or permit the formation of a third (distinct and/or effective) cutting edge 270c. Similarly, the junction or transition between side edge 260b and second major surface 230b (at second side 250b thereof) can comprise, accommodate, form, and/or permit the formation of a fourth (distinct and/or effective) cutting edge 270d.

As discussed in further detail below, those skilled in the art will also appreciate that once thickness 600 becomes sufficiently small, the transition between first major surface 230a and second major surface 230b, comprises, accommodates, forms, and/or permits the formation of a single cutting edge 270 in some embodiments. For example, as discussed further below, a thickness 600 less than or equal to about 0.254mm (0.01 inches) can comprise, accommodate, form, and/or permit the formation of a single cutting edge 270 in some embodiments.

Longitudinal side edges 260a and 260b can also be separated by a width 900. In at least one embodiment, width 900 can comprise the distance between first ends 240a, 240b and second ends 250a, 250b of first and second major surfaces 230a, 230b, respectively. Width 900 can comprise any suitable size, measurement, or value, including those greater than, less than, or equal to the size, measurement, or value of thickness 600 and/or length 500.

Moreover, body 200 can have a cross-sectional area 700 (e.g., disposed between first major surface 230a and second major surface 230b, and between first longitudinal side edge 260a and second longitudinal side edge 260b. One will appreciate, however, that in certain embodiments (e.g., embodiments having a single longitudinal side edge 260 or embodiments having more than two longitudinal side edges 260), cross-sectional area 700 can be differently disposed, bound, defined, and/or configured. Thus, first major surface 230a, second major surface 230b, and one or more longitudinal side edge 260 can at least partially bound a cross-sectional area 700. Cross-sectional area 700 can also have a cross-sectional height 800 (e.g., between first major surface 230a and second major surface 230b).

In some embodiments, the ratio of any of length 500, thickness 600, cross-sectional area 700, cross-sectional height 800, and/or width 900 to any other size, measurement, or value can vary without necessarily departing from the scope of this disclosure. In some embodiments, however, the ratio between two or more of the foregoing sizes, measurements, or values can be important to (or determinant of) one of more benefits of certain embodiments of the present disclosure. For instance, the ratio of cross-sectional area (A) 700 to the number of cutting edges (E) 270, in particular, can be unexpectedly important to one or more of the aforementioned advantages of certain embodiments of the present disclosure.

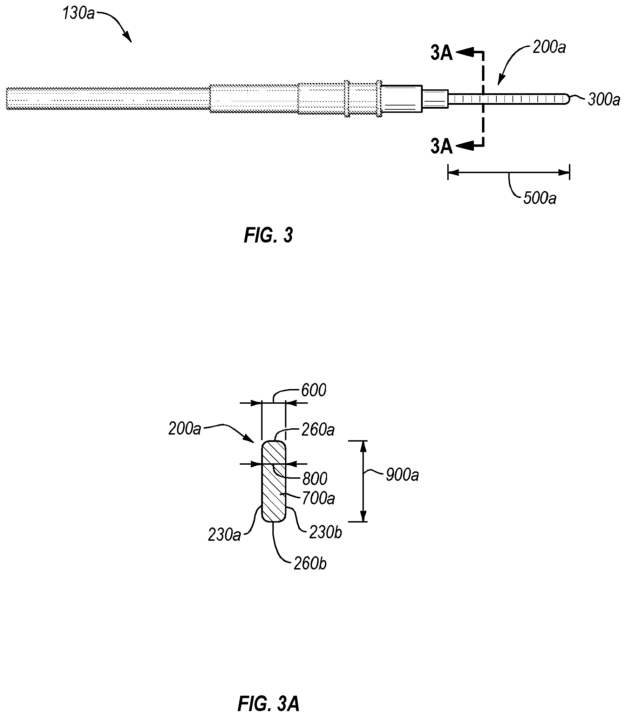

Turning now to Figures 3 and 3A, an electrosurgical electrode 130a, not according to the invention, can comprise a body 200a. Body 200a (or one or more components thereof) can have a length 500a. As illustrated in Figure 3A, body 200a can include first and second major surface 230a and 230b (e.g., separated by cross-sectional height 800), and first and second longitudinal side edges 260a and 260b (e.g., each having a thickness 600). In at least one embodiment, body 200a can have a width 900a and/or longitudinal side edges 260a and 260b can be separated by a width 900a. The measurement of width 900a (e.g., along with length 500a, cross-sectional height 800, and/or thickness 600) can contribute to the calculation of cross-sectional area 700a.

In some embodiments, width 900a can be smaller or less than width 900 (see Figure 2A). A smaller width 900a can permit, allow, and/or enable a higher level of precision in electrosurgical procedures. For instance, a user can limit the production of unwanted tissue damage or injury by reducing width 900 to a width 900a. Specifically, width 900a can allow a user to make a direction change during an electrosurgical procedure without damaging patient tissue to the same degree that would occur with width 900. In at least one embodiment, reduced width 900a can contribute to a reduction in cross-sectional area 700a and/or the size of tip 200a.

In at least one embodiment, thickness 600a (of side edge(s) 260a and/or 260b) can be greater than about 0.254 mm (0.01 inches). In addition, the cross-sectional area 700a-to-number of longitudinal cutting edges 270 ratio can be less than or equal to about 0.0004 square inches per longitudinal cutting edge (in²/E) (e.g., when longitudinal side edge(s) 260 are greater than about 0.254mm (0.01 inches). In an exemplary embodiment, an illustrative body 200a (having a substantially rectangular cross-section) can have a substantially uniform cross-sectional height 800 of about 0.0185 inches (0.4699 mm). Opposing longitudinal side edges 260a and 260b, separated by a width 900a of about 0.05 inches (1.27 mm), can also have thicknesses of about 0.0185 inches (0.4699 mm), respectively. Accordingly, body 200a can have four (4) longitudinal cutting edges 270 and a cross-sectional area 700a of 0.000925 square inches (in²) (0.596773 mm²). As a result, body 200a can have a cross-sectional area 700a-to-number of longitudinal cutting edges 270 ratio of 0.000231 square inches per longitudinal cutting edge (in²/E) (e.g., 0.1492 mm²/E).

One will appreciate that while additional sizes, measurements, values, and/or ratios are contemplated herein, in the foregoing embodiment, each must conform to the required parameters (i.e., a longitudinal side edge thickness greater than about 0.254mm (0.01 inches), and a cross-sectional area 700a-to-number of longitudinal cutting edges 270 ratio that is less than or equal to about 0.0004 in²/E. Thus, any combination of respective sizes, measurements, and/or values (e.g., for a longitudinal side edge thickness greater than about 0.254mm (0.01 inches), longitudinal side edge length, cutting edge length, body length, body width, body height, cross-sectional thickness, cross-sectional width, cross-sectional height, and/or cross-sectional area, etc.) resulting in a cross-sectional area-to-number of longitudinal cutting edges ratio that is less than or equal to about 0.0004 in²/E is contemplated herein.

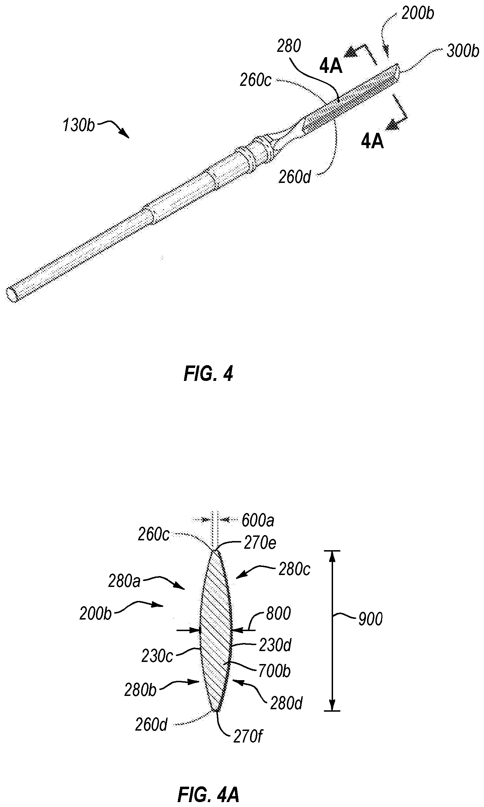

In one or more other embodiments, the body of the electrosurgical electrode can comprise at least one side edge having a thickness less than or equal to about 0.254mm (0.01 inches), and a cross-sectional area-to-number of longitudinal cutting edges ratio that is less than or equal to about 0.000150 in²/E. In at least one embodiment, a longitudinal side edge having a thickness less than or equal to about 0.254mm (0.01 inches) can be achieved by tapering at least a portion of the body from a cross-sectional height of greater than about 0.254mm (0.01 inches) to the longitudinal side edge. For instance, referring now to Figures 4 and 4A, electrosurgical electrode 130b according to the invention has a body 200b having side edges 260c and 260d, and can have a tip 300b, and at least one tapered portion 280.

As illustrated in Figure 4A, body 200b according to the invention has opposing major surfaces 230c and 230d, which transition into (or are continuous with) opposing side edges 260c and 260d. Importantly, body 200b includes tapered portion 280a extending from a portion of first major surface 230c to or toward first side edge 260c. Similarly, body 200b includes tapered portion 280b extending from a portion of first major surface 230c to or toward second side edge 260d. Likewise, body 200b includes tapered portion 280c extending from a portion of second major surface 230d to or toward first side edge 260c. Similarly, body 200b includes tapered portion 280d extending from a portion of second major surface 230d to or toward second side edge 260d.

In some embodiments, body 200b can have a height 800 (similar to or different than height 800 of body 200). Likewise, body 200b can have a width 900 (similar to or different than width 900 of body 200). For instance, width 900 can correspond to width 900a in some embodiments. Indeed, the actual (linear or curved) measurement of height 800; width 900, 900a; and/or thickness 600, 600a; can be or comprise any suitable value (e.g., within any range of values or combination of values sufficient to comply with the parameters (for cross-sectional area-to-number of longitudinal cutting edges ratio(s)) disclosed and/or described herein).

Furthermore, because of tapered portions 280, the surface length of major surface 230a and/or 230b from side edge 260c to side edge 260d can be greater or longer than the linear width of body 200b. Similarly, the thickness 600a of side edge(s) 260c, 260d can be less than height 800 of body 200b. Accordingly, the cross-sectional area 700b of body 200b can be less than the product of height 800 and width 900 in some embodiments. Those skilled in the art will also appreciate that reducing height 800 and/or width 900 can decrease cross-sectional area 700b in some embodiments.

Those skilled in the art will appreciate that once thickness 600a becomes sufficiently small, the transition(s) between first major surface 230c and second major surface 230d can form a single (effective) cutting edge 270e, 270f on one or both of side edges 260c, 260d. In other words, the junction between first major surface 230c and side edge 260c may not be surgically distinguishable over the junction between second major surface 230d and side edge 260c in some embodiments. Thus, side edge 260c, in effect, becomes or constitutes cutting edge 270e. The same can apply to side edge 260d, in which side edge 260d can become or constitute cutting edge 270f.

In one or more embodiments of the invention, body 200b comprises at least one side edge 260c, 260d having a thickness less than or equal to about 0.254mm (0.01 inches), and a cross-sectional area 700b-to-number of longitudinal cutting edges ratio that is less than or equal to about 0.000150 in²/E. In at least one embodiment, body 200b can have a cross-sectional height 800 greater than about 0.01 and/or at least one tapered portion 280. Thus, according to the invention, body 200b can comprise a lenticular cross-section (or cross-sectional shape or configuration) as illustrated in Figure 4A.

Furthermore, with a side edge thickness 600a less than or equal to about 0.254mm (0.01 inches), side edge 260c can produce and/or present (only) one (effective) longitudinal cutting edge 270e in certain embodiments. Similarly, side edge 260d, having a thickness 600a less than or equal to about 0.254mm (0.01 inches), can also produce and/or present (only) one (effective) longitudinal cutting edge 270f in certain embodiments. Thus, a body 200b having sided edges 260c and 260d with side edge thicknesses 600a less than or equal to about 0.254mm (0.01 inches) can comprise only two effective cutting edges 270e, 270f (e.g., as opposed to four effective cutting edges 270a, 270b, 270c, and 270d as in body 200 and/or 200a).

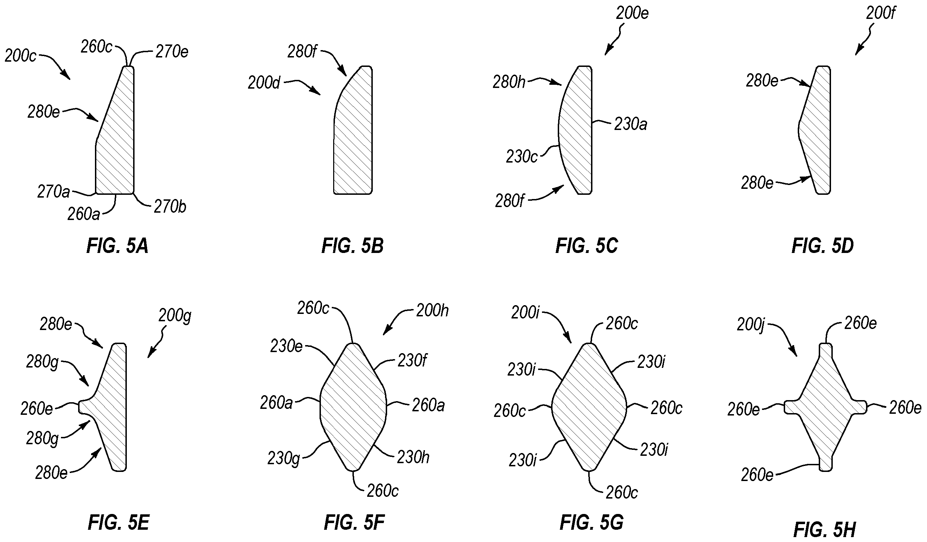

Figures 5A-5H illustrate some variations, not according to the invention, on the embodiments illustrated in the foregoing figures. Figure 5A illustrates a body 200c having a side edge 260a, a substantially straight or linear tapered portion 280e, and a side edge 260c. Accordingly, body 200c can have three effective cutting edges 270a, 270b, and 270e in some embodiments. Figure 5B illustrates a body 200d having a substantially curved or rounded tapered portion 280f. Figure 5C illustrates a body 200e having a substantially straight or linear first major surface 230a and a substantially curved or rounded second major surface 230c. Accordingly, body 200e (or second major surface 230c thereof) can include two tapered portions 280h. Figure 5D illustrates a body 200f having two substantially straight or linear tapered portions 280e.

In some embodiments, one or more major surfaces, side edges, and/or effective cutting edges can have an extended configuration. For instance, Figure 5E illustrates a body 200g having an extended or protruding side edge 260e. Furthermore, body 200g includes two inverted tapered portions 280g adjacent to extended or protruding side edge 260e and two substantially straight or linear tapered portions 280e extending from inverted tapered portions 280g. Figure 5F illustrates a body 200h having four major surfaces 230e, 230f, 230g, and 230h. Major surfaces 230e and 230f, as well as major surfaces 230g and 230h are separated by (or merge or transition into) side edges 260a. Major surfaces 230e and 230g, as well as major surfaces 230f and 230h are separated by (or merge or transition into) side edges 260c. Figure 5G illustrates a body 200i having four major surfaces 230i with intervening side edges 260c. Figure 5H illustrates a body 200j having extended or protruding side edges 260e.

Those skilled in the art will appreciate that sizes, shapes, configurations, transitions, and/or radii of curvatures can be modified based on the requirements of the surgeon or other user. For instance, an exemplary body illustrating side edge(s) 260c, for instance, can alternatively be configured with side edge(s) 260a, 260e, and/or any other side edge(s) disclosed and/or described herein. Exemplary illustration of a particular side edge is not necessarily limited to the illustrated side edge. Rather, any suitable combination of side edge(s), cutting edge(s), major surface(s), etc. is contemplated herein. Thus, side edge(s) appearing to correspond to a measurement of less than or equal to 0.254mm (0.01 inches) are not so limited. Instead, such side edge(s) can alternatively comprise measurement(s) greater than 0.254mm (0.01 inches) without necessarily departing from the scope of this disclosure.

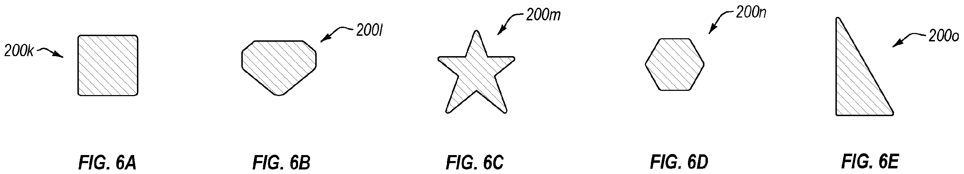

Figures 6A-6E illustrate a variety of geometric cross-sectional shapes and/or configurations not according to the invention. For instance, Figure 6A illustrates a body 200k having a substantially square cross-sectional configuration. Those skilled in the art will appreciate, however, that a wide variety of quadrilateral configurations can be implemented in various embodiments of the present disclosure. For instance, body 200k can also or alternatively comprise a rectangular, rhomboidal, trapezoidal, cross-sectional configuration. Parallelograms are also contemplated herein.

Other polygonal configurations (including concave, convex, regular, and irregular) are also contemplated herein, including a body 200l having a diamond-shaped cross section (Figure 6B), a body 200m having a star-shaped cross section (Figure 6C), a body 200n having a hexagonal-shaped cross section (Figure 6D), and a body 200o having a triangular-shaped cross section Figure 6E). One will also appreciate that pentagons, heptagons, octagons, and other polygonal configurations are also contemplated herein. Moreover, a triangular-shaped configuration can comprise an equilateral triangle, an isosceles triangle, a scalene triangle, a right triangle, an acute triangle, and/or an obtuse triangle.

Some embodiments can include various non-geometric, rounded, partially-rounded, or other shapes, forms, and/or configurations. For instance, Figure 7A illustrates a body 200p having an airfoil or tear-drop cross-section or cross-sectional configuration. Figure 7B illustrates a body 200q having a partial (e.g., half) airfoil or tear-drop cross-section. Figure 7C likewise illustrates a body 200r having a partial (e.g., half) airfoil or tear-drop cross-section. Figure 7D illustrates a body 200s having a lenticular cross-section according to the invention. Figure 7E illustrates a body 200t having a partial (e.g., half) lenticular cross-section. Figure 7F likewise illustrates a body 200u having a partial (e.g., half) lenticular cross-section. One will appreciate, however, that "partial" as used herein can also include a quarter, three-quarter, or other portion of a complete shape or configuration.

Figure 7G illustrates a body 200v having a partial (e.g., half) cone cross-section. Full cone and other similar configurations are also contemplated herein. Figure 7H illustrates a body 200w having a bi-lobe cross-section. Figure 7I illustrates a body 200x having a tri-lobe cross-section. One will appreciate that other multi-lobe configurations are also contemplated herein. Figure 7J illustrates a body 200y having a semi-circular cross-section. Figure 7K illustrates a body 200z having a round cross-section with one abutment. Figure 7L illustrates a body 200aa having a round cross-section with a plurality of abutments. Figure 7M illustrates a body 200bb having a ninja star cross-section. Figure 7N illustrates a body 200cc having an arrowhead cross-section. Figure 7O illustrates a body 200dd having a partial "yin-yang" cross-section (e.g., half yin-yang, etc.).

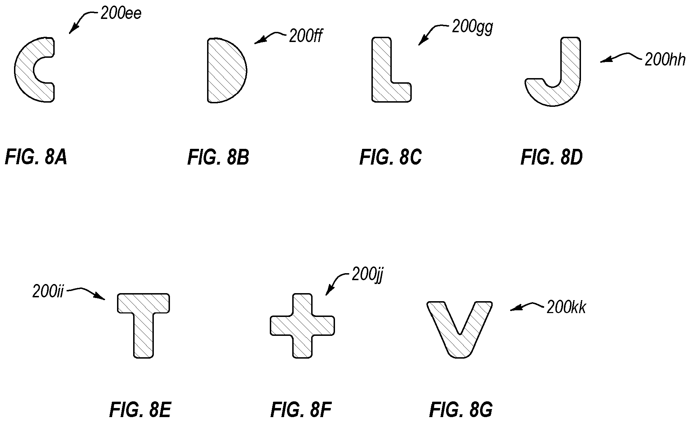

Various cross-sectional configurations can also adopt such shapes as Arabic, Roman, or other letters or written characters. For instance, Figure 8A illustrates a body 200ee having a "C"-shaped cross-section. Figure 8B illustrates a body 200ff having a "D"-shaped cross-section. Figure 8C illustrates a body 200gg having an "L"-shaped cross-section. Figure 8D illustrates a body 200hh having a "J"-shaped cross-section. Figure 8E illustrates a body 200ii having a "T"-shaped cross-section. Figure 8F illustrates a body 200jj having an "X"-shaped cross-section. Figure 8G illustrates a body 200kk having a "V"-shaped cross-section.

The cross-sectional configuration of one or more embodiments of the present disclosure can be uniform from the first end of the body to the second end of the body. Those skilled in the art will appreciate, however, that variable cross-sectional configurations are also contemplated herein. For instance, the tip of the body can be tapered in one or more embodiments, giving the body a variable cross-sectional configuration between the first end and the second end thereof. Furthermore, cross-sectional configuration can change along the length of the body without departing from the scope of this disclosure. Similarly, the length of the elongated body between the first end and the second end need not be perfectly or substantially planar and/or linear. For instance, curves, bends, and/or other changes in elongated shape are also contemplated herein.

The working ends of electrosurgical electrodes can be configured to provide great versatility in cutting and/or cauterizing tissue and/or blood vessels in a variety of different surgical procedures. Furthermore, the electrode tips can be configured to produce significantly improved performance in cutting efficiency, dramatic reduction in unwanted tissue damage, and improved post-operative recovery. For instance, each of the electrodes illustrated in the foregoing figures can include or be formed with one or more shaped or sharpened working edges. The shaped working edges can further concentrate the electrical energy transferred from the electrode to the patient's tissue. The further concentrated electrical energy can further reduce the amount of extraneous charge loss into surrounding tissue by increasing the incision speed, thereby reducing the activation time and level of thermal necrosis in tissues surrounding the incision site.

Similarly, each of the illustrated electrodes can be formed with a limited thickness, height, width, and/or mass to limit the amount of latent heat or thermal energy that can build up in the electrode. Reducing the amount of latent heat within the electrode can further reduce the amount of latent heat that is transferred from the electrode to the tissue, which reduces the amount of tissue damage caused in tissue surrounding the incision site.

Additionally, a non-stick coating can serve to eliminate or reduce the clinging of charred tissue to the blade, thereby reducing incidences of unwanted tissue damage. A non-stick material suitable for use as a coating can be, but is not limited to, PTFE or a hybrid material that can include a combination of at least one of an organic material and an inorganic material, and that provides the coated surface with desirable properties, such as a high temperature stability, flexibility, and a low temperature application condition so that the coating layer may be applied by a spray or dip process. An example of a hybrid coating is provided in U.S. Patent No. 6,951,559, entitled "Utilization of a Hybrid Material in a Surface Coating of an Electrosurgical Instrument" that issued on October 4, 2005 to Greep.

The foregoing examples and embodiments represent exemplary embodiments and are provided for illustrative purposes only. Accordingly, the disclosed examples and embodiments are meant to illustrate one or more aspects of the invention and are not intended to limit the scope of the present invention as defined by the appended claims. A variety of aspects compatible with and/or contemplated within the scope of one or more embodiments of the present disclosure can be found in U.S. Patent No. 5,496,315, U.S. Patent No. 5,697,926, U.S. Patent No. 5,893,849, U.S. Patent No. 6,039,735, U.S. Patent No. 6,039,735, U.S. Patent No. 6,066,137, U.S. Patent No. 8,439,910, and U.S. Patent No. 8,500727.

It is to be understood that this disclosure is not limited to parameters of the particularly exemplified products, processes, compositions, kits, and/or methods, which may, of course, vary. It is also to be understood that the terminology used herein is only for the purpose of describing particular embodiments of the present disclosure, and is not intended to limit the scope of the invention in any manner.

Additionally, the terms "including," "having," "involving," "containing," "characterized by," and variants thereof (e.g., "includes," "has," and "involves," "contains," etc.) as used herein, including in the claims, shall be inclusive and/or open ended, shall have the same meaning as the word "comprising" and variants thereof (e.g., "comprise" and "comprises"), and does not exclude additional, un-recited elements or method steps, illustratively.

It will be appreciated that where a range a values (e.g., less than, greater than, at least, or up to a certain value, or between two recited values) is disclosed or recited in connection with any of the embodiments described herein, any specific value or range of values falling within the disclosed range of values is likewise disclosed and contemplated herein. For instance, disclosure of a cross-sectional area-to-longitudinal cutting edges ratio less than or equal to 0.0004 in²/E, or between 0 and 0.0004 in²/E, includes, illustratively, a specific disclosure of: (i) a cross-sectional area-to-number of longitudinal cutting edges ratio of 0.0001 in²/E, 0.00025 in²/E, 0.000399 in²/E, 0.0004 in²/E, or any other value between 0 and 0.0004 in²/E; and/or (ii) a cross-sectional area-to-number of longitudinal cutting edges ratio between 0.00001 in²/E and 0.00035 in²/E, between 0.0002 in²/E and 0.0003 in²/E, between 0.00025 in²/E and 0.000275 in²/E, and/or any other range of values between 0 and 0.0004 in²/E, 1 inch = 2.54 cm.

Unless defined otherwise, all technical and scientific terms used herein have the same meaning as commonly understood by one of ordinary skill in the art to which the present disclosure pertains. While a number of methods and materials similar or equivalent to those described herein can be used in the practice of the present disclosure, only preferred materials and methods are described herein.

The described embodiments are to be considered in all respects only as illustrative and not restrictive. The scope of the invention is, therefore, indicated by the appended claims rather than by the foregoing description. While certain embodiments and details have been included herein and in the attached invention disclosure for purposes of illustrating the invention, it will be apparent to those skilled in the art that various changes in the products, processes, compositions, kits, and methods disclosed herein may be made without departing from the scope of the invention, which is defined in the appended claims. Various modifications that fall within the scope of the appended claims will be apparent to one skilled in the art.

## Claims

1. An electrosurgical electrode (130b) adapted for use in performing electrosurgical operative procedures, the electrode comprising:
an elongated body (200b) extending a longitudinal length between a first end and a second end, the body being formed of a conductive material and adapted to be electrically connected to an electrosurgical generator to facilitate communication of electrical energy from the generator to the electrode for communicating radio frequency electrical energy to patient tissue for performing electrosurgical operative procedures thereupon, the body having:
first and second longitudinal side edges (260c, 260d) extending between the first end and the second end arranged parallel to the longitudinal length of the electrode, at least one of the first and second longitudinal side edges having a thickness less than or equal to 0.254 mm (0.01 inches) and thereby constituting a longitudinal cutting edge (270e, 270f);
a first major surface (230c) and a second major surface (230d) opposite the first major surface, the first and second longitudinal side edges being disposed between the first major surface and the second major surface to define a lenticular cross-sectional area determined in a plane perpendicular to the longitudinal length, wherein the cross-sectional area has a cross-sectional width between the first and second longitudinal side edges less than or equal to 1.397 mm (0.055 inches); and
a cross-sectional area-to-number of longitudinal cutting edges ratio that is less than or equal to 0.096774 square mm (0.000150 square inches) per longitudinal cutting edge.

2. The electrode of claim 1, wherein the at least one of the first and second longitudinal side edges is continuous with the first major surface and the second major surface such that the longitudinal cutting edge thereof comprises a junction between the first major surface and the at least one of the first and second longitudinal side edges or between the second major surface and the at least one of the first and second longitudinal side edges.

3. The electrode of claim 1, further comprising a tip disposed at the first end of the body.

4. The electrode of claim 3, wherein the tip is continuous with one or more of the first major surface, the second major surface, and the first and second longitudinal side edges, the tip comprising a configuration selected from the group consisting of a blunt configuration, a pointed configuration, a rounded configuration, and an angled configuration.

5. The electrode of claim 1, further comprising an insulating coating covering at least a portion of the body.

6. The electrode of claim 5, wherein the insulating coating comprises a material selected from the group consisting of PTFE, silicone, ceramic, glass, chlorinated hydrocarbon, fluorinated hydrocarbon, diamond, a high temperature polymer, a hydrophilic polymer, and a capacitor dielectric, the insulating coating provided with a thickness sufficient to ensure transmission of the radio frequency electrical energy from the body to the tissue.

## Patentansprüche

1. Elektrochirurgische Elektrode (130b), ausgelegt zur Verwendung bei der Durchführung elektrochirurgischer Operationsverfahren, wobei die Elektrode umfasst:
einen langgestreckten Körper (200b), der in einer Längserstreckung zwischen einem ersten Ende und einem zweiten Ende verläuft, wobei der Körper aus einem leitfähigen Material gebildet ist und dafür ausgelegt ist, elektrisch mit einem elektrochirurgischen Generator verbunden zu werden, um Übertragung von elektrischer Energie von dem Generator zu der Elektrode zu ermöglichen, um elektrische Energie mit Radiofrequenz auf Patientengewebe zu übertragen, um elektrochirurgische Operationsverfahren daran durchzuführen, wobei der Körper aufweist:
erste und zweite längslaufende Seitenkanten (260c, 260d), die zwischen dem ersten Ende und dem zweiten Ende verlaufen und parallel zu der Längserstreckung der Elektrode angeordnet sind, wobei wenigstens eine von der ersten und der zweiten längslaufenden Seitenkante eine Dicke von kleiner als oder gleich 0,254 mm (0,01 Inch) aufweist und dadurch eine längslaufende Schneidkante (270e, 270f) bildet;
eine erste Hauptoberfläche (230c) und eine zweite Hauptoberfläche (230d) gegenüber der ersten Hauptoberfläche, wobei die erste und die zweite längslaufende Seitenkante zwischen der ersten Hauptoberfläche und der zweiten Hauptoberfläche angeordnet sind, um eine linsenförmige Querschnittsfläche, die in einer Ebene senkrecht zu der Längserstreckung bestimmt ist, zu definieren, wobei die Querschnittsfläche eine Querschnittsbreite zwischen der ersten und der zweiten längslaufenden Seitenkante von kleiner als oder gleich 1,397 mm (0,055 Inch) aufweist; und
ein Verhältnis von Querschnittsfläche zu Anzahl von längslaufenden Schneidkanten, das kleiner als oder gleich 0,096774 Quadrat-mm (0,000150 Quadratinch) pro längslaufender Schneidkante ist, aufweist.

2. Elektrode gemäß Anspruch 1, wobei die wenigstens eine von der ersten und der zweiten längslaufenden Seitenkante durchgehend mit der ersten Hauptoberfläche und der zweiten Hauptoberfläche ist, so dass die längslaufende Schneidkante davon eine Verbindung zwischen der ersten Hauptoberfläche und der wenigstens einen von der ersten und der zweiten längslaufenden Seitenkante oder zwischen der zweiten Hauptoberfläche und der wenigstens einen von der ersten und der zweiten längslaufenden Seitenkante umfasst.

3. Elektrode gemäß Anspruch 1, ferner umfassend eine Spitze, die an dem ersten Ende des Körpers angeordnet ist.

4. Elektrode gemäß Anspruch 3, wobei die Spitze mit einer oder mehreren von der ersten Hauptoberfläche, der zweiten Hauptoberfläche und der ersten und der zweiten längslaufenden Seitenkante durchgehend ist, wobei die Spitze eine Konfiguration ausgewählt aus der Gruppe bestehend aus einer stumpfen Konfiguration, einer spitzen Konfiguration, einer abgerundeten Konfiguration und einer gewinkelten Konfiguration aufweist.

5. Elektrode gemäß Anspruch 1, ferner umfassend eine isolierende Beschichtung, die wenigstens einen Teil des Körpers bedeckt.

6. Elektrode gemäß Anspruch 5, wobei die isolierende Beschichtung ein Material ausgewählt aus der Gruppe bestehend aus PTFE, Silicon, Keramik, Glas, chloriertem Kohlenwasserstoff, fluoriertem Kohlenwasserstoff, Diamant, einem Hochtemperaturpolymer, einem hydrophilen Polymer und einem Kondensatordielektrikum umfasst, wobei die isolierende Beschichtung mit einer Dicke bereitgestellt ist, die ausreicht, um Übertragung der elektrischen Energie mit Radiofrequenz von dem Körper auf das Gewebe zu gewährleisten.

## Revendications

1. Électrode électrochirurgicale (130b) adaptée pour être utilisée dans la réalisation de procédures opératoires électrochirurgicales, l'électrode comprenant :
un corps allongé (200b) s'étendant sur une longueur longitudinale entre une première extrémité et une seconde extrémité, le corps étant formé d'un matériau conducteur et adapté pour être connecté électriquement à un générateur électrochirurgical afin de faciliter la communication d'énergie électrique du générateur à l'électrode pour communiquer de l'énergie électrique radiofréquence au tissu du patient afin de réaliser des procédures opératoires électrochirurgicales sur celuici, le corps ayant :
des premier et second bords latéraux longitudinaux (260c, 260d) s'étendant entre la première extrémité et la seconde extrémité, agencées parallèlement à la longueur longitudinale de l'électrode, au moins l'un des premier et second bords latéraux longitudinaux ayant une épaisseur inférieure ou égale à 0,254 mm (0,01 pouce) et constituant ainsi un bord de coupe longitudinal (270e, 270f) ;
une première surface principale (230c) et une seconde surface principale (230d) opposée à la première surface principale, les premier et second bords latéraux longitudinaux étant disposés entre la première surface principale et la seconde surface principale pour définir une surface de section transversale lenticulaire déterminée dans un plan perpendiculaire à la longueur longitudinale, la surface de section transversale ayant une largeur de section transversale entre les premier et second bords latéraux longitudinaux inférieure ou égale à 1,397 mm (0,055 pouce) ; et
un rapport entre la surface de la section transversale et le nombre de bords de coupe longitudinaux inférieur ou égal à 0,096774 mm² (0,000150 pouce carré) par bord de coupe longitudinal.

2. Électrode selon la revendication 1, l'au moins un des premier et second bords latéraux longitudinaux étant continu avec la première surface principale et la seconde surface principale de telle sorte que le bord de coupe longitudinal de celle-ci comprend une jonction entre la première surface principale et l'au moins un des premier et second bords latéraux longitudinaux ou entre la seconde surface principale et l'au moins un des premier et second bords latéraux longitudinaux.

3. Électrode selon la revendication 1, comprenant en outre une pointe disposée au niveau de la première extrémité du corps.

4. Électrode selon la revendication 3, la pointe étant continue avec une ou plusieurs de la première surface principale, de la seconde surface principale et des premier et second bords latéraux longitudinaux, la pointe comprenant une configuration choisie dans le groupe constitué d'une configuration émoussée, d'une configuration pointue, d'une configuration arrondie et d'une configuration angulaire.

5. Électrode selon la revendication 1, comprenant en outre un revêtement isolant couvrant au moins une partie du corps.

6. Électrode selon la revendication 5, le revêtement isolant comprenant un matériau choisi dans le groupe constitué par le PTFE, le silicone, la céramique, le verre, un hydrocarbure chloré, un hydrocarbure fluoré, le diamant, un polymère haute température, un polymère hydrophile et un diélectrique de condensateur, le revêtement isolant ayant une épaisseur suffisante pour assurer la transmission de l'énergie électrique radiofréquence à partir du corps au tissu.
